# DEMANDE DE BREVET EUROPEEN

(11) **EP 1 837 392 A1**
(43) Date de publication de la demande: **26.09.2007**
(21) Numéro de dépôt: 06290451.1
(22) Date de dépôt: 21.03.2006
(51) Int. Cl.: C11B 9/02

(54) **Huile de magnolia champaca, son procédé d'obtention, et compositions la contenant**

(71) Demandeur: Chanel Parfums Beauté, 92200 Neuilly-sur-Seine (FR)
(72) Inventeur: Maestro, Yannick Gérard, 13500 Martiques (FR); Lasserre, Christelle Marie Suzanne, Jersey City, NJ07302 (US)
(74) Mandataire: Renard, Emmanuelle

(57) **Abrégé**

L'invention concerne une huile extraite de fleur d'au moins une espèce de Magnolia champaca, son procédé d'obtention, les compositions cosmétiques, pharmaceutiques ou dermatologiques la contenant, ainsi que son utilisation en tant qu'agent actif polyfonctionnel pour la prévention et/ou le traitement d'altérations de la peau.

## Description

L'invention concerne une huile extraite de fleur d'au moins une espèce de Magnolia champaca, son procédé d'obtention, une composition cosmétique, pharmaceutique ou dermatologique la contenant, ainsi que son utilisation en tant qu'agent actif polyfonctionnel dans une composition pharmaceutique, cosmétique ou dermatologique, pour la prévention et/ou le traitement d'altérations de la peau dus, notamment, au vieillissement ou à des mécanismes physiologiques liés au vieillissement ou à des troubles apparentés à ces mécanismes.

La peau est principalement constituée de trois couches, à savoir, en partant de la plus superficielle, l'épiderme, le derme et l'hypoderme.

La couche externe de la peau, l'épiderme, est stratifiée et contribue largement à assurer la protection de la peau vis-à-vis des agressions extérieures. Le derme est un tissu conjonctif assurant à la fois les fonctions de cohésion et de nutrition de la peau.

Le vieillissement cutané résulte de deux processus distincts et indépendants qui font intervenir des facteurs intrinsèques ou extrinsèques.

Le vieillissement intrinsèque ou chronobiologique correspond au vieillissement « normal » ou physiologique lié à l'âge.

Le vieillissement extrinsèque correspond au vieillissement provoqué d'une manière générale par l'environnement et plus particulièrement au photovieillissement dû à l'exposition au soleil.

La présente invention s'intéresse au vieillissement cutané intrinsèque ou physiologique ainsi qu'au vieillissement cutané extrinsèque.

Le vieillissement cutané est consécutif à une transformation des tissus conjonctifs et à l'abaissement de la capacité de régénération cellulaire. Cet effet se manifeste par l'apparition de ridules et de stigmates au cours du temps. La microcirculation diminue au niveau du derme superficiel. Les macromolécules telles que le collagène, l'élastine et les glycosaminoglycanes, dont l'acide hyaluronique est l'un des constituants, sont chimiquement modifiées. L'épaisseur même du derme régresse, les fibres sont dégradées et la peau perd ses propriétés biomécaniques et élastiques. Les phénomènes d'oxydation chimique et enzymatique augmentent avec l'âge et entraîne l'accroissement des réactions de pontage entre les fibres telles que les fibres de collagènes.

Les altérations associées au vieillissement peuvent se manifester de différentes manières, parmi lesquelles on peut citer :
- une désorganisation des fibres d'élastine entraînant une perte de fermeté, de souplesse et d'élasticité ou par l'apparition de télangiectasies;
- la perte d'éclat due à la réduction de la microcirculation et à un ralentissement du renouvellement cellulaire au niveau de l'épiderme et l'apparition de fines rides ou ridules ;
- le jaunissement de la peau qui développe un aspect parcheminé accompagné de l'apparition de taches pigmentaires associées à un dysfonctionnement de la synthèse de la mélanine (ou mélanogénèse) ;
- la sécheresse cutanée résultant d'une diminution de la fonction barrière de la couche cornée et à un ralentissement du renouvellement épidermique.

Il existe, de ce fait, un besoin de fournir un agent actif polyfonctionnel susceptible d'agir sur un ensemble de causes d'altérations de la peau dues au vieillissement et/ou à une modification des mécanismes physiologiques liés au vieillissement ou apparentés.

La plante Magnolia champaca, également dénommée dans la littérature Michelia champaca, est un arbuste appartenant à la famille des Magnoliacées, originaire des régions humides et boisées de Chine. L'essence de ses fleurs est couramment utilisée en parfumerie.

La demande JP 05-207777 décrit une composition cosmétique comprenant un extrait de Michelia champaca obtenu par extraction à l'aide d'un solvant polaire et son utilisation en tant agent antioxydant.

Des compositions contenant un extrait de Michelia champaca L., ayant une activité blanchissante de la peau, ou une activité de capteur d'oxygène actif, ou une activité antibactérienne, sont décrites dans la demande JP 10-269482.

La demande JP 2003-299743 concerne des compositions anti-UV absorbant les rayons UVA et UVB et comprenant un extrait aqueux d'une plante de Michelia champaca.

L'activité anti-inflammatoire et antipyrétique d'extraits méthanoliques de Michelia champaca L. (variété blanche) est décrite dans R.Vimala et al., Indian J. Exp. Biol., 1997, 35, 1310-1314.

Les auteurs de la présente invention ont maintenant mis en évidence, de façon tout à fait surprenante, qu'une huile extraite de fleur d'au moins une espèce de Magnolia champaca, présentait, par le biais de stimulation ou d'inhibition de mécanismes physiologiques, des activités susceptibles d'agir sur les symptômes dus au vieillissement, ou à des mécanismes physiologiques liés au vieillissement, ou à des troubles apparentés à ces mécanismes au niveau de l'épiderme et/ou du derme.

Cette constatation a conduit à la mise au point de nouvelles compositions cosmétiques, pharmaceutiques ou dermatologiques, plus particulièrement utiles pour toutes les applications dans lesquelles on cherche à agir sur les symptômes dus au vieillissement, ou sur les mécanismes physiologiques liés au vieillissement, ou sur les troubles apparentés à ces mécanismes au niveau de l'épiderme et/ou du derme.

Par « huile », on entend un produit obtenu par extraction à l'aide d'un solvant apolaire et très riche en composés insolubles dans l'eau.

L'invention concerne donc, selon un premier aspect, une huile extraite de fleur d'au moins une espèce de Magnolia champaca, caractérisée en ce qu'elle comprend :
- 20 à 70% d'ester benzoïque
- 15 à 35% d'acides gras saturés et/ou insaturés,
lesdites proportions étant exprimées en pourcentages relatifs par rapport à l'ensemble des constituants séparés par chromatographie en phase gazeuse.

De préférence, l'huile selon l'invention provient de Magnolia champaca, variété « gold », également dénommée « fleur d'or ».

Par « teneur exprimée en pourcentages relatifs par rapport à l'ensemble des constituants séparés par chromatographie en phase gazeuse », on entend que la teneur en chacun des constituants est déterminée par rapport à l'ensemble des constituants séparés par le système chromatographique. Seuls sont présents les composés extraits par le solvant lors de la préparation d'échantillon et pouvant se vaporiser dans l'injecteur.

L'échantillon doit être préparé selon la norme NF T 60-233 de mai 1977 « Préparation des esters méthyliques d'acides gras », (§ 5.2 - Méthode applicable aux corps gras acides et acides gras. Les conditions chromatographiques sont décrites dans la norme NF EN ISO 5508 de juin 1995.

Brièvement, la méthode consiste à estérifier les acides gras en milieu méthanolique acide, puis à les extraire avec de l'heptane, puis à injecter la solution heptanique en chromatographie en phase gazeuse.

De préférence, l'huile selon l'invention comprend :
- de 10 à 40% de benzoate de cinnamyle,
- de 5 à 30% de benzoate de phényléthyle,
- de 5 à 30% de benzoate de benzyle,
- de 5 à 30% d'acide linoléique,
- de 2 à 20 % d'acide linolénique.
lesdites proportions étant exprimées en pourcentages relatifs par rapport à l'ensemble des constituants séparés par chromatographie en phase gazeuse.

L'invention concerne également, selon un aspect ultérieur, un procédé de préparation d'une huile de Magnolia champaca tel que définie plus haut, comprenant au moins une étape de distillation moléculaire.

La matière première mise en oeuvre consiste, par exemple, en des fleurs fraîchement cueillies (fleurs fraîches) d'au moins une espèce de Magnolia champaca, de préférence Magnolia champaca variété « gold », que l'on peut broyer ou réduire en morceaux de manière usuelle.

Les fleurs sont soumises à une extraction par au moins un solvant apolaire, par exemple choisi parmi l'hexane, le cyclohexane, l'heptane, l'isooctane, le CO₂ supercritique ou le dichlorométhane.

L'extraction est généralement réalisée en immergeant ou en agitant doucement les fleurs dans un ou plusieurs des solvants cités ci-dessus à des températures allant, par exemple, de la température ambiante à 100°C, pendant une durée d'environ 30 min à 12 h.

Après filtration, l'extraction peut être renouvelée plusieurs fois. Les solutions sont rassemblées et filtrées, afin d'éliminer les parties insolubles des fleurs. On élimine également, le cas échéant, le solvant s'il s'agit d'un solvant volatile tel que, par exemple, l'hexane ou le l'hexane, le cyclohexane.

Cette étape d'extraction est usuelle dans le domaine des extraits de plantes, et l'homme du métier est à même d'en ajuster les paramètres réactionnels, sur la base de ses connaissances générales.

Selon un aspect avantageux de l'invention, on peut effectuer une étape de fractionnement afin de purifier le produit obtenu à l'issue de l'extraction.

On utilisera, de préférence, un solvant ou un mélange de solvants choisi(s) parmi les alcools en C₁-C₄ tels que par exemple le méthanol, l'éthanol, l'isopropanol, etc., les solvants organiques tels que par exemple le propylène glycol, le dipropylène glycol etc., ou encore l'acétate d'éthyle, l'hexane, le cyclohexane ou tout autre solvant usuel dans le domaine.

Un fractionnement par fluide supercritique, de préférence par CO₂ supercritique, peut également être utilisé.

Comme précédemment, si un ou plusieurs solvants volatiles est (sont) utilisé(s), il y aura lieu de le ou les éliminer avant de passer à l'étape suivante.

A l'issue de l'extraction, et, éventuellement, de l'étape de fractionnement mentionnée ci-dessus, on obtient une concrète de Magnolia champaca. Ce type d'extrait est habituellement utilisé en tant que produit intermédiaire dans la fabrication d'absolues destinées à l'industrie de la parfumerie.

Avantageusement, ladite concrète est soumise à une étape de distillation moléculaire, réalisée dans un appareil de distillation à film raclé et à court trajet.

Selon un aspect préféré, l'invention concerne donc un procédé d'obtention d'une huile de Magnolia champaca telle que défini plus haut, comprenant les étapes consistant à :
- procéder à une extraction à partir de fleurs fraîches d'au moins une espèce de Magnolia champaca à l'aide d'au moins un solvant apolaire,
- soumettre ledit extrait à au moins une étape de distillation moléculaire, et
- récupérer le distillat.

Pour effectuer l'étape de distillation moléculaire, les distillateurs moléculaires à film raclé et à court trajet sont préférés. Ils comprennent une chambre de distillation dotée d'un racleur tournant, permettant l'étalement en continu sur la surface d'évaporation (surface chaude) de produit à distiller. Les vapeurs de produit sont condensées par le biais d'un doigt réfrigéré, placé au centre de la chambre de distillation. La récupération des résidus et distillat se fait par écoulement gravitationnel. Cette technique a pour objectif de séparer les constituants des mélanges complexes en tirant profit de leurs différents points d'ébullition. La distillation moléculaire à film raclé et à court trajet a pour avantage de réduire la température de distillation car la distillation s'effectue sous un vide poussé et de réduire également le temps de séjour du mélange à séparer dans le distillateur. En effet, la vitesse de décomposition des produits s'accroît énormément en fonction de la température et du temps d'exposition et dans un distillateur de type « alambic » par exemple, les mélanges peuvent séjourner pendant des heures à des températures importantes, ce qui entraîne une dénaturation.

Jusqu'à maintenant, dans le cas des huiles végétales, un tel procédé n'avait été utilisé que pour isoler des fractions insaponifiables ou purifier ces mêmes huiles végétales en éliminant les fractions insaponifiables, ou encore, dans le cas de fractions hydrosolubles ou liposolubles, pour décolorer ou désodoriser les extraits.

Or, on a maintenant trouvé que, de manière surprenante, l'utilisation d'une étape de distillation moléculaire permettait d'isoler une fraction huileuse à partir d'un extrait liposoluble dans lequel cette fraction huileuse est en quantité minoritaire.

Des conditions préférentielles de mise en oeuvre du procédé sont données ci-après :
Avantageusement, on ajoute à la concrète de Magnolia champaca obtenue après extraction et éventuellement, fractionnement, avant la distillation, un solvant lourd choisi parmi les huiles minérales ou végétales et les polyols, tels que par exemple du polyéthylène glycol, pour faciliter la distillation ou l'écoulement le long de la colonne.

Le mélange de la concrète obtenue après extraction et éventuellement, fractionnement et du solvant lourd est introduit à débit constant, à une température comprise entre 20 et 120°C, de préférence entre 50 et 100°C, sur la paroi chaude d'un évaporateur cylindrique.

Par raclage au moyen de balais rotatifs à bagues, il est étalé en film mince sur toute la surface de la paroi chaude maintenue à une température comprise entre environ 100 et 200°C, et, de préférence entre 110 et 170°C.

Au contact de celle-ci, et sous la très faible pression régnant dans l'évaporateur, de l'ordre de 10 mbar à 0,001 mbar, le produit volatile est alors partiellement et progressivement vaporisé, alors que le produit moins volatile coule le long de la paroi.

Les vapeurs émises sont condensées sur la paroi froide concentrique de la paroi chaude et placée à très courte distance de celle-ci, de préférence à une température comprise entre 40 et 120°C, notamment entre 60 et 100°C.

Les produits séparés au cours de l'opération s'écoulent par gravité le long des parois chaude et froide.

On récupère le distillat que l'on soumet, le cas échéant, à une opération supplémentaire de séparation (filtration ou centrifugation, par exemple).

On obtient ainsi une huile de Magnolia champaca selon l'invention.

Avantageusement, ladite huile est de couleur claire. De plus, ladite huile est dépourvue de solvant ou de tout autre réactif chimique étant intervenu au cours de son extraction.

Egalement, ladite huile se présente sous une forme suffisamment concentrée pour pouvoir être utilisée sans entraîner les problèmes de formulation habituellement liés aux concentrations nécessaires pour obtenir une activité dans les compositions cosmétiques, pharmaceutiques ou dermatologiques sous forme d'émulsion, et sans présenter une couleur foncée, contrairement aux extraits végétaux obtenus par des procédés usuels, lorsqu'ils sont sous forme concentrée.

De ce fait, l'huile selon l'invention peut être utilisée directement pour la préparation d'une composition cosmétique, pharmaceutique ou dermatologique.

L'invention concerne également l'huile de Magnolia champaca susceptible d'être obtenue par le procédé décrit plus haut.

Selon un aspect ultérieur, l'invention concerne l'utilisation d'une huile de Magnolia champaca tel que décrite plus haut, en tant qu'agent actif polyfonctionnel dans une composition cosmétique, pharmaceutique ou dermatologique, pour la prévention et/ou le traitement d'altérations de la peau dus au vieillissement ou à des mécanismes physiologiques liés au vieillissement ou à des troubles apparentés à ces mécanismes.

En particulier, l'invention concerne l'utilisation d'une huile de Magnolia champaca telle que décrite plus haut dans une composition cosmétique , en tant qu'agent améliorant la réparation cellulaire ou la cicatrisation et/ou agent d'activation du métabolisme des cellules de l'épiderme, et/ou agent de piégeage des radicaux libres et/ou agent de détoxification de l'épiderme.

L'invention concerne également l'utilisation d'un extrait de d'une huile de Magnolia champaca telle que décrite plus haut en tant qu'agent améliorant la réparation cellulaire ou la cicatrisation et/ou agent d'activation du métabolisme des cellules de l'épiderme, et/ou agent de piégeage des radicaux libres et/ou agent de protection de l'ADN nucléaire et/ou agent de protection de l'ADN mitochondrial et/ou agent de détoxification de l'épiderme, pour la préparation d'une composition pharmaceutique ou dermatologique.

En effet, de manière avantageuse, on a trouvé que l'extrait de vanille selon l'invention possédait plusieurs activités d'intérêt vis-à-vis de mécanismes physiologiques préventifs ou réparateurs liés aux altérations de la peau, dues notamment au vieillissement.

L'invention concerne donc, plus particulièrement, l'utilisation d'une huile de Magnolia champaca telle que décrite plus haut en tant qu'agent inhibiteur de la synthèse de mélanine, notamment en tant qu'agent inhibiteur de la synthèse d'endothéline, dans une composition cosmétique, pharmaceutique ou dermatologique. L'endothéline est une protéine messager qui favorise la prolifération des mélanocytes et l'accroissement de la mélanogenèse, responsable de la pigmentation de la peau liée au vieillissement (« taches brunes »).

L'invention concerne, en particulier, l'utilisation d'une huile de Magnolia champaca telle que décrite plus haut en tant qu'agent inhibiteur du facteur de transcription MITF (pour « Microphtalmia-associated Transcription Factor»), un facteur de régulation et de différenciation des mélanocytes, dans une composition cosmétique ou dermatologique.

On a également trouvé que, de manière avantageuse, l'huile de Magnolia champaca selon l'invention stimule la synthèse de plusieurs familles de facteurs de croissance par les kératinocytes.

En particulier, cette activité s'exerce vis-à-vis des facteurs de croissance suivants :
- Le PDGF ou facteur de croissance dérivé des plaquettes (en anglais « platelet-derived growth factor »), qui exerce, notamment, une activité mitogène sur la plupart des cellules dérivées du mésenchyme (Lepisto J et al, 1995, Biochem. Biophys. Res. Commun, 209(2):393-9) et stimule la synthèse de collagène et de collagènase par ces cellules, jouant ainsi un rôle dans des processus physiologiques tels que la cicatrisation et la réparation tissulaire (Tan EM et al, Biochem. J. 1995, 310 (Pt 2):585-8) ; il a également été montré que le niveau d'induction des PDGF et la capacité réplicative des cellules étaient liés à l'âge : des fibroblastes sénescents ont leur taux de PDGF qui diminue (Karlsson C et al., J. Cell Physiol., 1994, 158(2):256-62). Egalement, il a été montré que la quantité et le type des différents facteurs de croissance pouvait expliquer les différences de capacité des tissus à se réparer avec l'âge. (Ashcroft GS et al., J. Anat, 190, 1997, (Pt 3):351-65).
- le VEGF ou facteur de croissance endothélial vasculaire (en anglais « vascular endothelial growth factor ») qui représente dans la peau un facteur majeur de l'angiogénèse cutanée. L'épiderme est une source importante de VEGF, secrété en grande quantité par les kératinocytes en prolifération. L'ARNm du VEGF est exprimé par les kératinocytes normaux, à la fois dans un tissu *in situ* et en culture cellulaire. Il a été montré que le VEGF maintiendrait l'homéostasie des cellules endothéliales et leur capacité à répondre à une stimulation angiogénique, même chez le sujet âgé (Watanabe Y et al., 1997, Oncogene 14: 2025-2032).
   D'autre part une diminution du VEGF a été observée suite à une exposition aux radiations UV, (Mildner M et al., Photochem. Photobiol., 1999; 70(4):674-9).
- le HB-EGF ou facteur de croissance épidermique se liant à l'héparine (en anglais « heparin-binding epidermal growth factor »), qui joue un rôle important dans la régulation et la différenciation des kératinocytes (Iwamoto et al., Cytokine and Growth Factors Rewiews, 2000, 11:335-344), ainsi que dans la sénescence de cellules jeunes dont la croissance dépend de ce facteur (JID Suppl.24: S46-S50 ; Kanzaki Y et al., Exp. Cell. Res., 2002, 279(2):321-329);

Par « activateur de la synthèse de plusieurs familles de facteurs de croissance», on entend selon l'invention, toute molécule capable de stimuler l'activité, et/ou l'expression et/ou la libération d'au moins l'une de ces facteurs de croissance exprimé et/ou synthétisé par et dans les kératinocytes.

L'invention concerne donc l'utilisation d'une huile de Magnolia champaca telle que décrite plus haut en tant qu'agent activateur de la synthèse d'au moins un facteur de croissance cellulaire par les kératinocytes, notamment en tant qu'agent activateur d'au moins un facteur de croissance choisi parmi PDGF, VEGF et HB-EGF, dans une composition cosmétique, pharmaceutique ou dermatologique.

L'invention concerne également, l'utilisation une huile de Magnolia champaca telle que décrite plus haut en tant qu'agent inhibiteur de l'activité des enzymes impliqués dans la dégradation de la matrice, en particuliers les métalloprotéinases de la matrice (dénommées MMP pour « matrix metalloprotease »), et tout particulièrement les MMP 3 (ou stromélysine 1) et MMP9 (ou gélatinase B).

Les métalloprotéinases matricielles sont des enzymes (endoprotéases) qui dégradent la matrice extracellulaire dans le cadre du remodelage physiologique de la peau. L'âge et l'exposition aux irradiations UV de type A et/ou B ont pour conséquence d'augmenter l'activité de ces MMP, en particulier celle de la MMP3 (ou stromélysine 1) et de la MMP9 (ou gélatinase-élastase B) et cette augmentation contribue, avec le ralentissement de la croissance cellulaire, au vieillissement chronologique de la peau (WO 98/36742). De ce fait, la matrice extracellulaire est dégradée de façon accrue, avec pour résultante l'affaissement des tissus de la peau et la formation de rides (Rittie L et al., Ageing Res. Rev., 2002, 1 (4) :705-20 ; Chung JH et al., J. Invest. Dermatol, 2001, 117(5):1218-24).

Par « inhibiteur de métalloprotéinase », on entend selon l'invention, toute molécule capable d'inhiber l'activité, et/ou l'expression et/ou la libération d'au moins l'une des métalloprotéinases exprimée et/ou synthétisée par et dans la peau.

L'invention concerne également, selon des aspects préférés, l'utilisation d'une huile de Magnolia champaca telle que décrite plus haut dans une composition cosmétique ou pour la préparation d'une composition pharmaceutique ou dermatologique, en tant qu'agent antioxydant.

L'invention concerne également, selon des aspects préférés, l'utilisation d'une huile de Magnolia champaca telle que décrite plus haut dans une composition cosmétique ou pour la préparation d'une composition pharmaceutique ou dermatologique, en tant qu'agent stimulant du métabolisme cellulaire.

L'invention concerne également, selon des aspects préférés, l'utilisation d'une huile de Magnolia champaca telle que décrite plus haut dans une composition cosmétique ou pour la préparation d'une composition pharmaceutique ou dermatologique, en tant qu'agent de détoxification de l'épiderme suite à un stress oxydatif, notamment par stimulation de la synthèse de DT diaphorase (NQO1) qui catalyse la détoxification des quinones et, également, par stimulation de la synthèse de la gluthatione S-transférase qui catalyse la détoxification des métabolites chimiques, tels que les produits de proxidation lipidiques.

L'invention concerne également, selon un autre aspect préféré, l'utilisation une huile de Magnolia champaca telle que décrite plus haut en tant dans une composition cosmétique ou pour la préparation d'une composition pharmaceutique ou dermatologique, en tant qu'agent stimulateur de l'énergie cellulaire en particulier du cytochrome C et de la pAMP kinase.

Le Cytochrome C oxidase, étape finale de la chaine de transport des électrons, est crucial pour la respiration cellulaire et contribue également à la synthèse d'ATP cellulaire (Lalla A et al., West Indian Med J. 2001 Jun; 50(2): 111-6. Le statut énergétique de la cellule est coordonné par le système de la protéine kinase activée par l'AMP (AMPK) et contribue également au contenu en ATP de la cellule (Dagon Y,et al., J Biol Chem. 2005, 23; 280(51):42142-8).

L'invention concerne également, selon un aspect ultérieur, une composition cosmétique ou une composition pharmaceutique ou dermatologique comprenant une huile de Magnolia champaca telle que décrite plus haut et un véhicule cosmétiquement ou pharmaceutiquement acceptable.

De préférence, ledit extrait est présent dans la composition cosmétique, pharmaceutique ou dermatologique à raison de 0,001 à 10% en poids total de la composition, en particulier à raison de 0,01 à 5%, de préférence de 0,1 à 1% en poids total de la composition.

Ladite composition cosmétique pharmaceutique ou dermatologique peut notamment, être adaptée à une application par voie topique.

Avantageusement, ladite composition cosmétique, pharmaceutique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau dans huile ou huile dans eau.

La composition cosmétique, pharmaceutique ou dermatologique selon l'invention comprend également un solvant choisi en fonction des différents ingrédients et de la forme d'administration.

A titre d'exemples, on peut citer l'eau (de préférence de l'eau déminéralisée), un alcool tel que l'éthanol, ou un éther de diéthylène glycol tel que l'éthoxydiglycol ou l'éther monométhylique de diéthylène glycol.

Ladite composition cosmétique, pharmaceutique ou dermatologique peut également comprendre au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent chélatant, un agent actif, une poudre organique ou inorganique, un pigment, un agent autobronzant, un filtre solaire, une huile essentielle et un parfum.

Notamment, de façon non limitative, ladite composition peut contenir :
- Un ou plusieurs agent(s) humectant(s), choisi(s), par exemple, parmi les polyols tels que la glycérine, le propylène glycol, le butylène glycol, le polyéthylène glycol, les sucres ou leurs dérivés, l'urée, l'acide glycolique ou ses sels, l'acide lactique ou ses sels, l'acide hyaluronique.
   Ledit agent humectant sera présent dans la composition à une teneur de l'ordre de 0,01 à 20%, de préférence 0,5 à 10% en poids total de la composition.
- Un ou plusieurs agent(s) émollient(s), choisi(s), par exemple, parmi les silicones volatiles ou non volatiles (diméthicone, cyclométhicone, diméthiconol, et apparentées), les hydrocarbonés à longues chaînes, les esters d'acides gras ou d'alcools gras.
   Ledit agent émollient sera présent dans la composition à une teneur de l'ordre de 0,5 à 30%, de préférence 0,5 à 15% en poids total de la composition.
- Un ou plusieurs agent(s) gélifiants(s) et/ou épaississant(s) de préférence un ou plusieurs gélifiants hydrophiles, choisi(s), par exemple, parmi les dérivés cellulosiques, gommes d'origine végétale (guar, caroube, alginates, carraghenanes, pectine), d'origine microbienne (xanthane), les argiles (laponite), les polymères carbovinyliques (carbomer), les copolymères acryliques tels que les copolymères d'acrylates/alkylacrylates, les polyacrylamides, les ammonium acryloyldiméthyltaurate copolymères tels que l'ammonium acryloyldiméthyltaurate/vinylpyrolidone (VP) copolymer et l'ammonium acryloyldiméthyl-taurate/beheneth-25 méthacrylate copolymer (tel que par exemple ceux vendus sous les dénominations Aristoflex AVC et HMB de Clariant).
   Des agents gélifiants non filmogènes peuvent être par exemple choisis parmi les argiles (laponite), les ammoniums acryloyldiméthyltaurate/vp copolymer et ammonium acryloyldiméthyltaurate/beheneth-25 méthacrylate copolymer (tel que par exemple ceux vendus sous les dénominations Aristoflex AVC et HMB de Clariant).
   Ledit agent gélifiant et/ou épaississant sera présent dans la composition à une teneur de l'ordre de 0 à 10% en poids total de la composition, de préférence 0,1 à 5% en poids total de la composition.
- Un ou plusieurs agent(s) tensioactif(s) ou co-tensioactif(s), de préférence non ionique, présent dans une teneur de l'ordre de 0,01 à 10%, de préférence 0,1 à 5% en poids total de la composition.
- Un ou plusieurs corps gras liquide(s) ou solide(s) à température ambiante, communément dénommé(s) huiles(s), volatile(s) ou non volatile(s), hydrocabroné(s), siliconé(s), linéaire(s), cyclique(s) ou ramifié(s), beurre(s), par exemple, l'isododécane, le cyclopentadimethylsiloxane, la diméthicone, l'isononanoate d'isononyle, le pentaerythrityl tetraisostéarate, des triglycérides d'acide caprylique et caprique, le beurre de karité, etc., de préférence à raison de 0,01 à environ 15%, de préférence 0,1 à 10% en poids total de la composition.
- Un ou plusieurs agent(s) actif(s) d'origine naturelle, biotechnologique ou synthétique ayant une activité biologique et ayant une efficacité sur la peau via des sites biologiques, par exemple choisi(s) parmi les vitamines, les oligoéléments, l'allantoïne, les protéines végétales, les extraits végétaux, etc. En particulier, ledit agent actif peut être un extrait d'algue.
- Un ou plusieurs colorant(s) hydrosoluble(s) tels que, par exemple, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine ou la xanthophylle de préférence à raison de 0 à environ 2% en poids total de la composition.

D'autres additifs habituellement utilisés en cosmétique, pharmacie ou dermatologie peuvent également être présents dans la composition selon l'invention, notamment des conservateurs, des agents chélatants, des agents antioxydants, des huiles essentielles ou des parfums bien connus dans le domaine technique.

L'homme du métier est en mesure de choisir, parmi l'ensemble de ces éventuels additifs, aussi bien la composition que la quantité de ceux qui seront ajoutés à la composition, de telle sorte que celle-ci conserve l'ensemble de ses propriétés.

L'invention est illustrée de manière non limitative par les exemples ci-dessous.

### Exemple 1 : procédé de préparation d'une huile de Magnolia champaca selon l'invention

### 1) Extraction hexanique

On charge 200 kg de fleurs fraîches de Magnolia champaca dans un réacteur de 1000 l.

On ajoute 800 l d'hexane et on chauffe à 35°C pendant 40 min.

Le réacteur est vidangé de la solution hexanique. Le solvant chargé est conservé.

600 I d'hexane sont à nouveau chargé dans le réacteur. On chauffe pendant 40 min à 35°C.

La solution hexanique est à nouveau vidangée. Les deux filtrats sont rassemblés.

L'hexane est évaporée.

On récupère ainsi 0,56 kg de concrète de Magnolia champaca.
Rendement : 0,28%

### 2) Distillation moléculaire

303 g de la concrète sont mélangés à 130 g de polyéthylèneglycol 600 (Dénomination INCl: PEG12) :

On effectue deux passages dans un appareil de distillation de type KDL4 (UIC GmH) selon les paramètres de distillation suivants :

| **Paramètres de distillation** | ***1^{er} passage*** | ***2^{nd} passage*** |
|---|---|---|
| Débit d'insertion (ml/h) | 400 | 400 |
| Température évaporateur (°C) | 135 | 145 |
| Température condenseur (°C) | 70 | 70 |
| Température d'introduction du produit (°C) | 75 | 75 |
| Agitation (tours/min) | 180 | 180 |
| Pression vide (mbar) | 10 | 7x10⁻² |

Les rendements de la distillation sont les suivants, exprimés sur le poids de matière première :
Distillat 1^{er} passage = 25,7 %
Distillat 2^{nd} passage = 31,34 %

### 3) Mélange

Après rassemblement et homogénéisation des deux distillats, un mélange est réalisé avec du propylène glycol : 90% en masse de propylène glycol avec 10% en masse du distillat. Le mélange est homogénéisé et filtré à 25 µm. Le filtrat est récupéré.

On obtient 1640 g d'huile de Magnolia champaca.

### Exemple 2: analyse de la composition d'une huile de Magnolia champaca selon l'invention

100 mg d'extrait obtenu lors de l'étape 3 du procédé décrit dans l'exemple 1 sont solubilisés dans 2 ml d'une solution méthanolique d'acide chlorhydrique 1 N. La solution est placée après agitation au bain-marie à 80°C pendant 10 min.

L'échantillon ainsi estérifié est récupéré par extraction liquide/liquide avec 2 ml d'heptane et injecté dans la chromatographie en phase gazeuse.

La chromatographie est réalisée dans les conditions suivantes :
Chromatographe en phase gazeuse (Agilent série 6890).
Injecteur avec ou sans division à 250°C.
Injection de 0,5µl d'échantillon préparé avec une division de 1/100^{ème}.
Séparation sur colonne capillaire 100 % polydiméthylsiloxane greffée, longueur 30 m, diamètre interne 0,25 mm, épaisseur de film : 0,25µm (Agilent série DB-1)
Gaz vecteur Hélium à 1 ml/min
Température initiale du four colonne 70°C
Gradient de température de 70°C à 250°C à 2°C/minutes et isotherme à 250°C pendant 60 min.
Durée totale : 120 min.
Détection : détecteur à ionisation de flamme (gaz auxiliaire azote).

Les pourcentages donnés dans le tableau 1 ci-dessous sont exprimés en pourcentages relatifs obtenus par l'intégrateur du chromatographe. Seuls les composés pouvant se vaporiser dans l'injecteur sont présents dans ces résultats. Le propylène glycol n'a pas été intégré et n'est donc pas listé dans le tableau ci-dessous.

L'identification des constituants a été réalisée par spectrométrie de masse.

Les résultats sont donnés dans le tableau 1 ci-dessous.

**Tableau 1**

| **TR (min)** | **Constituants** | **%GC relatifs** |
|---|---|---|
| 8,8 | Alcool benzylique | 0,28 |
| 10,9 | Oxyde de linalol | 2,87 |
| 11,3 | Benzoate de méthyle | 5,51 |
| 15,2 | Epoxy linalol | 2,09 |
| 25,1 | Eugénol | 4,37 |
| 33 | Tiglate de benzyle | 0,46 |
| 46,7 | Benzoate de benzyle | 12,19 |
| 51,1 | Benzoate de phényléthyle | 12,28 |
| 56,4 | Palmitate de méthyle | 7,43 |
| 61,3 | Benzoate de cinnamyle | 21,65 |
| 63,7 | Linoléate de méthyle | 11,41 |
| 63,8 | Linolénate de méthyle | 5,91 |
| 64,4 | Oléate de méthyle | 1,41 |
| | TOTAL | 87,86 |

### Exemple 3: étude de l'activité d'huile de Magnolia champaca selon l'invention vis-à-vis du facteur de croissance HB-EGF

On a testé, à différentes concentrations, un échantillon d'huile de Magnolia champaca obtenue à l'exemple 1, préalablement dilué à 10% dans du propylèneglycol.

### 1/ Préparation des cultures de kératinocytes

Ce protocole est commun à tous les essais d'activité biologique.

Des kératinocytes dérivés de prépuces néonataux (Clonetics, San Diego, USA) ont été ensemencés dans des plaques à 6 puits et cultivés dans du milieu de culture pour la croissance des kératinocytes (KBM, Clonetics), à savoir un milieu de culture modifié supplémenté en EGF humain recombinant, insuline, hydrocortisone, extrait hypophysaire bovin, gentamycine et amphotéricine b.

Après 24 h de culture dans un étuve à 37°C, 5% CO₂ et à saturation d'humidité les cellules sont lavées avec du tampon PBS pH 7,4 (Gibco) et incubées avec du milieu spécifique basique (KBM, Clonetics) contenant les produits à tester, pendant 6h ou 24h selon les tests, à différentes concentrations.

Chaque concentration a été testée en triple exemplaire sur deux donneurs pour les tests dits « ELISA » et « Western Blot », et en double exemplaire sur deux donneurs pour les tests de biologie moléculaire.

Un contrôle sans produit à tester (dit « non traité ») est également réalisé en gardant les cellules dans le même milieu sans traitement.

Un contrôle positif est éventuellement réalisé avec un produit de référence pour l'activité testée.

### 2/ Mesure de l'expression de l'ARN messager (ARNm) du HB-EGF

### Principe de l'essai :

On utilise l'amplification en chaîne par polymérase en temps réel (en anglais RT-PCR pour « Real Time-Polymerase Chain Reaction ») pour quantifier l'expression de l'ARN messager du HB-EGF dans un échantillon traité par rapport à un échantillon non traité. Les résultats sont normalisés par rapport à l'expression de gènes domestiques dans ces échantillons et corrigés en ce qui concerne les différences d'efficacité de PCR.

Les résultats sont exprimés en nombre de fois d'augmentation ou de diminution d'expression du gène cible (HB-EGF) dans l'échantillon traité, et non en nombre absolu de copies.

Les séquences des ADNc/ARNm des gènes investigués ont été obtenues chez GenBank.
Gène cible : HB-EGF
Gène domestique :PBDG

Toutes les amorces de PCR ont été obtenues en utilisant le logiciel PRIMER3 du Whitehead Institute for Biomedical Resarch.

### Protocole de l'essai :

L'ARNm a été isolé en utilisant le réactif Trizol (Invitrogen) selon les recommandations du fabricant. La transcription inverse a été effectuée à l'aide du kit gene Amp RNA PCR (Applied Biosystems) selon les recommandations du fabricant.

La mesure de PCR en temps réel a été effectuée en utilisant la technologie LightCycler (Roche Applied Science) avec détection SYBR Green I. Dans tous les essais, l'ADNc a été amplifié en utilisant un programme standardisé. Chaque capillaire LightCycler a été chargé avec 1,5 µl de DNA Master Mix, 1,8 µl de MgCl₂ (25mM), 10,1 µl d'eau et 0,5 µl de chaque amorce (10µM stock) . La quantité finale d'ADNc par réaction correspondait à 25 ng d'ARN total utilisé pour la transcription inverse.

La spécificité de la PCR a été testée par électrophorèse sur gel d'agarose et séquençage, et a été évaluée pour chaque échantillon en utilisant une analyse de point de fusion incluse dans le programme de PCR.

La quantification relative de l'expression du gène cible a été réalisée en utilisant le modèle mathématique de Pfaffl (Pfaffl, MW, Nucleic Acids Res. 29(9), p. E45, 2001).

Les résultats sont donnés dans le tableau 2 ci-dessous.

On a utilisé comme contrôle positif le facteur alpha de nécrose des tumeurs (TNFa) qui est une cytokine anti-inflammatoire connue pour stimuler le HB-EGF.

**Tableau 2**

| Concentration (%) | Activité HB-EGF |
|---|---|
| 0 (contrôle non traité) | 1 |
| Contrôle positif TNFa (100ng/ml) | 4,2 |
| 0,01 % | 14,2 |
| 0,005% | 8 |

Les résultats montrent que l'huile de Magnolia champaca selon l'invention possède une activité stimulatrice sur la synthèse d'ARNm de HBEGF.

### Exemple 4 : étude de l'activité d'huile de Magnolia champaca selon l'invention vis-à-vis du facteur de croissance VEGF

La mesure a été réalisée par RT-PCR de la même manière que dans l'exemple 3. Les séquences des ADNc/ARNm des gènes investigués ont été obtenues chez GenBank.
Gène cible : VEGF
Gène domestique : β2-microglobuline

Les conditions de culture des kératinocytes sont telles que décrites dans l'exemple 3.

Les résultats sont rapportés dans le tableau 3 ci-dessous:

**Tableau 3**

| Concentration (%) | Activité VEGF |
|---|---|
| 0 (contrôle non traité) | 1 |
| 0,01 % | 2,2 |

Les résultats montrent que l'huile de Magnolia champaca selon l'invention possède une activité stimulatrice sur la synthèse d'ARNm de VRGF.

### Exemple 5 : étude de l'activité d'huile de Magnolia champaca selon l'invention vis-à-vis du facteur de croissance PDGF-AA

L'évaluation quantitative des concentrations du facteur de croissance PDGF-AA humain dans les cultures de cellules a été effectuée à l'aide de la méthode ELISA en utilisant le kit d'essai immunologique Quantikine^{®} (n° DAA00, R&D Systems) .

Les conditions de culture des kératinocytes et les échantillons testés sont tels que décrits dans l'exemple 3.

Les résultats sont rapportés dans le tableau 4 ci-dessous:

**Tableau 4**

| Concentration (%) | % Activité PDGF-AA |
|---|---|
| 0 (contrôle non traité) | 100 |
| 0,01% | 230,15 |
| 0,005% | 307,2 |

Les résultats montrent que l'huile de Magnolia champaca selon l'invention possède une activité stimulatrice sur la synthèse de PDGF-AA.

### Exemple 6: étude de l'activité d'huile de Magnolia champaca selon l'invention vis-à-vis de l'endothéline-1

L'évaluation quantitative des concentrations d'endothéline-1 humaine dans les cultures de cellules a été effectuée à l'aide de la méthode ELISA en utilisant le kit d'essai immunologique Quantikine^{®} (n° BBE5, R&D Systems).

Les conditions de culture des kératinocytes et les échantillons testés sont tels que décrits dans l'exemple 3.

Les résultats sont rapportés dans le tableau 5 ci-dessous:

**Tableau 5**

| Concentration (%) | % Activité Endothéline-1 |
|---|---|
| 0 (contrôle non traité) | 100 |
| 0,01 % | 65,6 |
| 0,005% | 39,5 |

Les résultats montrent que l'huile de Magnolia champaca selon l'invention possède une activité inhibitrice sur la synthèse d'endothéline-1.

### Exemple 7: étude de l'activité d'huile de Magnolia champaca selon l'invention vis-à-vis du MITF

L'évaluation quantitative des concentrations en ARNm du MITF dans les cultures de cellules a été effectuée selon le protocole décrit dans l'exemple 3.
Gène cible : MITF
Gène domestique : β2-Microglobuline.

L'acide kojique a été utilisé en tant que contrôle positif de l'inhibition. Les résultats sont rapportés dans le tableau 6 ci-dessous.

**Tableau 6**

| Concentration (%) | Activité MITF |
|---|---|
| 0 (contrôle non traité) | 1 |
| Contrôle positif acide kojique (500µg/ml) | 0,55 |
| 0,01% | 0,21 |

Les résultats montrent que l'huile de Magnolia champaca selon l'invention possède une activité inhibitrice sur l'expression de l'ARNm du MITF.

### Exemple 8: étude de l'activité d'huile de Magnolia champaca selon l'invention vis-à-vis des métalloprotéinases de la matrice (MMP)

### 1/ Principe de l'essai

Les produits à tester sont incubés avec les MMP activées L'activité enzymatique est contrôlée par l'addition d'un substrat enzymatique fluorescent spécifique de chaque MMP.

### 2/ Protocole de l'essai

La pro-MMP3 ou la pro-MMP9 sont incubées dans les plaques avec une solution d'APMA (acétate p-aminophénylmercurique) 1 mM à température ambiante, sous légère agitation, pendant 1 h.

Différentes concentrations des différents inhibiteurs potentiels sont ajoutées. On incube ensuite le mélange à température ambiante sous légère agitation. La réaction enzymatique est provoquée en ajoutant le substrat fluorescent (SIGMA) solubilisé dans du DMSO.

La réaction enzymatique est suivie pendant 1 h à l'aide d'un spectrofluorimètre et la fluorescence est mesurée à une longueur d'onde d'excitation de 360 nm et une longueur d'onde d'émission de 460 nm pour MMP3 (ou stromélysine 1) ; à une longueur d'onde d'excitation de 320 nm et une longueur d'onde d'émission de 405 nm pour MMP9 (ou gélatinase-élastase B).

Les résultats sont exprimés en unité de fluorescence émise (RFU) qui représente la quantité de substrat hydrolysé par min.

Le spectrofluorimètre Microwin 2000 calcule la variation d'absorbance A, qui représente la vitesse initiale (Vi) de la réaction enzymatique. Dans chaque essai, on fait la moyenne des 3 valeurs de Vi obtenues pour chaque concentration d'inhibiteur potentiel. Les résultats de 10 expériences ont été exprimés en moyenne ± SD, puis présentés en pourcentage d'activité résiduelle.

Les résultats sont rapportés dans les tableaux 7 (MMP3 ou stromélysine 1) et 8 (MMP9 ou gélatinase B) ci-dessous.

On a utilisé comme contrôle positif l'acide éthylène diaminotétraacétique (EDTA), qui possède une activité inhibitrice bien connue sur la classe des métalloprotéinases.

**Tableau 7**

| Concentration (%) | % Activité MMP3 |
|---|---|
| 0 (contrôle non traité) | 100 |
| Contrôle positif EDTA 5mM | 0 |
| 0,01% | 75 |

**Tableau 8**

| Concentration (%) | % Activité MMP9 |
|---|---|
| 0 (contrôle non traité) | 100 |
| Contrôle positif EDTA 5mM | 0 |
| 0,01% | 65 |

Les résultats montrent que l'huile de Magnolia champaca selon l'invention inhibe l'activité des métalloprotéinases de la matrice MMP3 et MMP9.

### Exemple 9: étude de l'activité d'huile de Magnolia champaca selon l'invention vis-à-vis de la synthèse de la DT diaphorase :

Après avoir préparé les cellules selon le protocole de l'exemple 3, les cellules sont lysées pour leur analyse en Western Blot. Après la quantification de protéines selon le kit (Pierce Chemical Co, NY), les protéines sont fractionnées sur gradient de gel. Les membranes en difluorure de polyvinylidène (PVDF) issues du transfert des gels sont incubées en présence d'un anticorps anti-NQ01 polyclonal (Genetex, San Antonio, TX). Après rinçage les membranes sont révélées par un kit de détection « Supersignal West Dura » (Pierce Chemical Co. NY) et les bandes sont quantifiées par analyse d'image.

Les résultats sont rapportés dans le tableau 9 ci-dessous :

**Tableau 9**

| Concentration (%) | % Activité DT diaphorase |
|---|---|
| 0 (contrôle non traité) | 100 |
| 0,02% | 176 |
| 0,04% | 213 |

Les résultats montrent que l'huile de Magnolia champaca selon l'invention stimule l'activité de la DT diaphorase.

### Exemple 10: étude de l'activité d'huile de Magnolia champaca selon l'invention vis-à-vis de la synthèse de la Glutathione S transférase (GST):

Après avoir préparé les cellules selon le protocole de l'exemple 3, les cellules sont lysées et les surnageant récupérés. L'activité de la GST est évaluée par l'utilisation du kit « GST Activity Assay Kit » (Biodivision, Mountain View, CA) selon le protocole décrit par le fournisseur du kit.

Les résultats sont rapportés dans le tableau 10 ci-après :

**Tableau 10**

| Concentration (%) | % Activité GST |
|---|---|
| 0 (contrôle non traité) | 100 |
| 0,02% | 131 % |
| 0,04% | 120% |

Les résultats montrent que l'huile de Magnolia champaca selon l'invention stimule la synthèse de la Glutathione S transférase.

### Exemple 11: étude de l'activité d'huile de Magnolia champaca selon l'invention vis-à-vis de la synthèse du cytochrome C cellulaire :

La quantité de cytochrome C dans les lysats des kératinocytes préparés selon le protocole de l'exemple 3 est quantifiée avec le kit « Cytochrome C ELISA Kit » (Zymed^{®}).

Les résultats sont rapportés dans le tableau 11 ci-dessous :

**Tableau 11**

| Concentration (%) | % Activité Cytochrome C |
|---|---|
| 0 (contrôle non traité) | 100 |
| 0,01% | 132% |
| 0,02% | 147% |

Les résultats montrent que l'huile de Magnolia champaca selon l'invention stimule la synthèse du cytochrome C cellulaire.

### Exemple 12: étude de l'activité d'huile de Magnolia champaca selon l'invention vis-à-vis de la synthèse de l'AMP kinase :

Après avoir préparé les cellules selon le protocole de l'exemple 3, les cellules sont lysées pour leur analyse en Western Blot. Après la quantification de protéines selon le kit (Pierce Chemical Co, NY), les protéines sont fractionnées sur gradient de gel. Les membranes issues du transfert des gels sur membrane (PVDF) sont incubées en présence d'un anticorps monoclonal anti-AMPK ou phospho-AMPK a (Cell Signalling Technology, Beverly, MA). Apres rinçage les membranes sont révélées par le kit de détection « Supersignal West Dura » (Pierce Chemical Co. NY) et les bandes sont quantifiées par analyse d'image.

Les résultats sont rapportés dans le tableau 12 ci-dessous :

**Tableau 12**

| Concentration (%) | % Activité AMP kinase |
|---|---|
| 0 (contrôle non traité) | 100 |
| 0, 01 % | 231% |

Les résultats montrent que l'huile de Magnolia champaca selon l'invention possède une activité stimulatrice sur la synthèse de l'AMP kinase.

### Exemple 13: étude de l'activité antioxydante d'huile de Magnolia champaca selon l'invention

### Principe des trois protocoles

Les différents kits de tests antioxydants ABEC^{®}1 (Knight Scientific Limited) permettent de mesurer la capacité d'un échantillon à bloquer les radicaux libres. Dans chaque expérience, un type particulier de radical ou d'oxydant est généré : radical hydroxyle, oxydants halogénés ou superoxyde. Ces tests chimioluminescents contiennent une photoprotéine Pholasin^{®} unique qui émet de la lumière en présence de radicaux libres et de certains oxydants. La capacité antioxydante de l'échantillon est exprimée par le pourcentage de réduction du pic de luminescence de la Pholasin^{®} observé durant le temps des différents tests en présence ou en absence de l'échantillon ou des contrôles.

Pour chacune des études a, b et c ci-dessous, un contrôle positif a été mis en oeuvre.

### a/ étude de l'activité d'huile de Magnolia champaca selon l'invention vis-à-vis l'inhibition du radical hydroxyle :

On a utilisé comme contrôle positif le mannitol qui présente des effets anti-radicalaires connus vis à vis du radical hydroxyle.

**Tableau 13**

| Concentration (%) | % inhibition du radical hydroxyle |
|---|---|
| 0 (contrôle non traité) | 0 |
| Contrôle positif Mannitol 400mM | 90 |
| 0,1 % | 99 |
| 0,01 % | 88 |

Les résultats montrent que l'huile de Magnolia champaca selon l'invention possède une activité antioxydante envers le radical hydroxyle.

### b/ étude de l'activité d'huile de Magnolia champaca selon l'invention vis-à-vis l'inhibition des oxydants halogénés :

On a utilisé comme contrôle positif l'albumine sérique de bovin (BSA) qui présente une activité inhibitrice connue vis-à-vis des oxydants halogénés.

**Tableau 14**

| Concentration (%) | % inhibition des oxydants halogénés |
|---|---|
| 0 (contrôle non traité) | 0 |
| Contrôle positif BSA 1mg/ml | 88 |
| 0,1% | 87 |
| 0,01% | 43 |

Les résultats montrent que l'huile de Magnolia champaca selon l'invention possède une activité antioxydante envers les oxydants halogénés.

### c/ étude de l'activité d'huile de Magnolia champaca selon l'invention vis-à-vis l'inhibition du radical superoxyde :

On a utilisé comme contrôle positif la vitamine C (acide ascorbique). L'acide ascorbique est bien connu pour son rôle dans la formation de radicaux superoxydes.

**Tableau 15**

| Concentration (%) | % inhibition de radical superoxyde |
|---|---|
| 0 (contrôle non traité) | 0 |
| Contrôle positif Ascorbate 80µM | 74 |
| 0,1% | 82 |

Les résultats montrent que l'huile de Magnolia champaca selon l'invention possède une activité antioxydante envers le radical superoxyde.

### Exemple 14 : crème pour le contour des yeux comprenant une huile de Magnolia champaca selon l'invention

Les différents ingrédients mentionnés dans les tableaux 16 et 17 suivants sont dénommés par leur dénomination chimique usuelle ou selon la nomenclature internationale INCI (International Cosmetic Ingredient Dictionary and Handbook publié par The Cosmetic, Toiletry and Fragrance Association, 9th Edition, 2002).

**Tableau 16**

| **Ingrédients** | **Pourcentage en poids** |
|---|---|
| Eau déminéralisée | QSP 100 |
| **Phase grasse** | |
| Cetyl phosphate | 0,25 |
| Steareth-21 | 0,81 |
| Glyceryl stearate | 3,53 |
| Stearyl alcohol & cetyl alcohol | 1 |
| Oleyl alcohol | 2 |
| Isostearyl & isostearyl alcohol | 2,82 |
| Shea butter | 7,5 |
| Dicaprylate/caprate | 3,25 |
| **Phase aqueuse** | |
| Pentylene glycol | 2 |
| Propylene glycol | 5 |
| Glycerin | 7 |
| Glycerin & PEG-8 & caprylyl glycol | 7 |
| Sodium hyaluronate | 1 |
| Polyacrylamide | 2 |
| Sodium Acryloyldimethyl Taurate Copolymer | 1 |
| Carbomer | 0,35 |
| Xanthan gum | 0,10 |
| **Ingrédients actifs** | |
| Huile de Magnolia champaca obtenue selon l'exemple 1 | 0,10 |
| Nanoelespher FRE | 5,5 |
| Biopeptide (gly-his-lys) + Rigin (gly-gln-pro-arg) | 3 |
| Scenedesmus extract | 1 |
| Ronacare Bisabolol | 0,25 |
| Licorice PT40 | 0,4 |
| Trihydroxypalmitamidohydroxypropyl myristyl ether | 0,02 |
| Rhodiola extract | 1 |
| Phospholipids & Beta-sitosterol | 0,5 |
| Hamamelis | 5 |
| Prunus amygdalus dulcis (sweet almond) seed extract | 4 |

### Exemple 15: crème pour le visage comprenant une huile de Magnolia champaca selon l'invention

**Tableau 17**

| **Ingrédients** | **Pourcentage en poids** |
|---|---|
| Eau déminéralisée | QSP 100 |
| **Phase grasse** | |
| Cetyl phosphate | 2 |
| Steareth-21 | 0,40 |
| Glyceryl stearate | 3,33 |
| Cetyl alcohol | 0,6 |
| Oleyl alcohol | 3 |
| Isostearyl & isostearyl alcohol | 7 |
| Caprylic/capric acid triglycerides | 5 |
| Dimethicone | 3 |
| **Phase aqueuse** | |
| Propylene glycol | 7,5 |
| Sodium hayluronate | 1 |
| Carbomer | 0,35 |
| **Ingrédients actifs** | |
| Huile de Magnolia champaca obtenue selon l'exemple 1 | 0,10 |
| Nanoelespher FRE | 10 |
| Biopeptide (gly-his-lys) + Rigin (gly-gln-pro-arg | 3 |
| Scenedesmus extract | 1 |
| Ronacare Bisabolol | 0,25 |
| Licorice PT40 | 0,4 |
| Trihydroxypalmitamidohydroxypropyl myristyl ether | 0,02 |

## Revendications

1. Huile extraite de fleur d'au moins une espèce de Magnolia champaca, **caractérisée en ce qu'**elle comprend :
- 20 à 70% d'ester benzoïque
- 15 à 35% d'acides gras saturés et/ou insaturés,
lesdites proportions étant exprimées en pourcentages relatifs par rapport à l'ensemble des constituants séparés par chromatographie en phase gazeuse.

2. Huile selon la revendication 1, **caractérisé en ce qu'**elle provient de Magnolia champaca, variété « gold ».

3. Huile selon la revendication 1 ou 2, **caractérisée en ce qu'**elle comprend :
- de 10 à 40% de benzoate de cinnamyle,
- de 5 à 30% de benzoate de phényléthyle,
- de 5 à 30% de benzoate de benzyle,
- de 5 à 30% d'acide linoléique,
- de 2 à 20 % d'acide linolénique.
lesdites proportions étant exprimées en pourcentages relatifs par rapport à l'ensemble des constituants séparés par chromatographie en phase gazeuse.

4. Huile selon l'une quelconque des revendications 1 à 3, **caractérisé en ce qu'**elle est dépourvue de solvant.

5. Huile selon l'une quelconque des revendications 1 à 4, **caractérisé en ce qu'**elle est dépourvue de réactif chimique étant intervenu au cours de son extraction.

6. Procédé de préparation d'une huile de Magnolia champaca selon l'une quelconque des revendications 1 à 5, **caractérisé en ce qu'**il comprend au moins une étape de distillation moléculaire.

7. Procédé de préparation d'une huile de Magnolia champaca selon l'une quelconque des revendications 1 à 5, **caractérisé en ce qu'**il comprend les étapes consistant à :
- procéder à une extraction à partir d'un broyat de fleurs fraîches d'au moins une espèce de Magnolia Champaca à l'aide d'au moins un solvant apolaire,
- soumettre ledit extrait à au moins une étape de distillation moléculaire, et
- récupérer le distillat.

8. Procédé selon la revendication 7, **caractérisé en ce que** ledit solvant apolaire est choisi parmi l'hexane, le cyclohexane, l'heptane, l'isooctane, le CO₂ supercritique ou le dichlorométhane.

9. Procédé selon l'une quelconque des revendications 6 à 8, **caractérisé en ce que** l'étape de distillation moléculaire est effectuée à une température comprise entre environ 100 et 200°C et à une pression comprise entre environ 10 et 0,001 mbar.

10. Procédé selon l'une quelconque des revendications 7 à 9, **caractérisé en ce que**, à l'issue de l'extraction, on effectue un fractionnement à l'aide d'au moins un solvant choisi parmi les alcools en C₁-C₄, les polyols, l'acétate d'éthyle, l'hexane, le cyclohexane et le CO₂ supercritique.

11. Procédé selon l'une quelconque des revendications 6 à 10, **caractérisé en ce que**, préalablement à la distillation moléculaire, on ajoute un solvant choisi parmi les huiles minérales ou végétales et les polyols.

12. Procédé selon l'une quelconque des revendications 6 à 11, **caractérisé en ce que** la température d'introduction dans la colonne de distillation est comprise entre 20 et 120°C, de préférence entre 50 et 100°C.

13. Procédé selon l'une quelconque des revendications 6 à 12, **caractérisé en ce que** la température de condensation est comprise entre 40 et 120°C, de préférence entre 60 et 100°C.

14. Huile de Magnolia champaca, susceptible d'être obtenue par le procédé selon l'une quelconque des revendications 6 à 13.

15. Utilisation d'une huile de Magnolia champaca selon l'une quelconque des revendications 1 à 5 ou 14 en tant qu'agent actif polyfonctionnel dans une composition pharmaceutique, cosmétique ou dermatologique, pour la prévention et/ou le traitement d'altérations de la peau dus au vieillissement ou à des mécanismes physiologiques liés au vieillissement ou à des troubles apparentés à ces mécanismes.

16. Utilisation selon la revendication 15 d'une huile de Magnolia champaca selon l'une quelconque des revendications 1 à 5 ou 14 dans une composition cosmétique, en tant qu'agent améliorant la réparation cellulaire ou la cicatrisation et/ou agent d'activation du métabolisme des cellules de l'épiderme et/ou agent de piégeage des radicaux libres et/ou agent de régénération de l'épiderme et/ou agent de détoxification de l'épiderme.

17. Utilisation selon la revendication 15 d'une huile de Magnolia champaca selon l'une quelconque des revendications 1 à 5 ou 14 en tant qu'agent améliorant la réparation cellulaire ou la cicatrisation et/ou agent d'activation du métabolisme des cellules de l'épiderme et/ou agent de piégeage des radicaux libres et/ou agent de protection de l'ADN nucléaire et/ou agent de protection de l'ADN mitochondrial et/ou agent de régénération de l'épiderme et/ou agent de détoxification de l'épiderme, pour la préparation d'une composition pharmaceutique.

18. Utilisation selon la revendication 15 d'une huile de Magnolia champaca selon l'une quelconque des revendications 1 à 5 ou 14 en tant qu'agent améliorant la réparation cellulaire ou la cicatrisation et/ou agent d'activation du métabolisme des cellules de l'épiderme et/ou agent de piégeage des radicaux libres et/ou agent de protection de l'ADN nucléaire et/ou agent de protection de l'ADN mitochondrial et/ou agent de régénération de l'épiderme et/ou agent de détoxification de l'épiderme, pour la préparation d'une composition dermatologique.

19. Utilisation selon l'une quelconque des revendications 15 à 18 d'une huile de Magnolia champaca en tant qu'agent inhibiteur de la synthèse de mélanine.

20. Utilisation selon l'une quelconque des revendications 15 à 19 d'une huile de Magnolia champaca en tant qu'agent inhibiteur de la synthèse d'endothéline ou agent inhibiteur du facteur de transcription MITF.

21. Utilisation selon l'une quelconque des revendications 15 à 19 d'une huile de Magnolia champaca en tant qu'agent activateur de la synthèse d'au moins un facteur de croissance cellulaire par les kératinocytes.

22. Utilisation selon la revendication 21, **caractérisée en ce que** ledit facteur de croissance est choisi parmi PDGF, VEGF et HB-EGF.

23. Utilisation selon l'une quelconque des revendications 15 à 18 d'une huile de Magnolia champaca en tant qu'un agent inhibiteur de l'activité des métalloprotéinases de la matrice MMP3 (ou stromélysine 1) et MMP9 (ou gélatinase-élastase B).

24. Utilisation selon l'une quelconque des revendications 15 à 18 d'une huile de Magnolia champaca en tant qu'un agent stimulateur du métabolisme cellulaire.

25. Utilisation selon la revendication 24, **caractérisée en ce que** ledit facteur de stimulation du métabolisme cellulaire est choisi parmi le cytochrome C ou l'AMP kinase.

26. Utilisation selon l'une quelconque des revendications 15 à 18 d'une huile de Magnolia champaca en tant qu'agent antioxydant.

27. Utilisation selon l'une quelconque des revendications 15 à 18 d'une huile de Magnolia champaca en tant qu'agent de détoxification de l'épiderme, par stimulation de la synthèse de DT diaphorase.

28. Composition cosmétique, pharmaceutique, ou dermatologique, **caractérisée en ce qu'**elle comprend une huile de Magnolia champaca selon l'une quelconque des revendications 1 à 5 ou 14 et un véhicule cosmétiquement ou pharmaceutiquement acceptable.

29. Composition cosmétique, pharmaceutique, ou dermatologique selon la revendication 28, **caractérisée en ce que** ladite huile est présente dans la composition pharmaceutique, cosmétique ou dermatologique à raison de 0,001 à 10% en poids total de la composition.

30. Composition cosmétique, pharmaceutique ou dermatologique selon la revendication 29, **caractérisée en ce que** ladite huile est présente dans la composition pharmaceutique, cosmétique ou dermatologique à raison de 0,01 à 5%, de préférence de 0,1 à 1 % en poids total de la composition.

31. Composition cosmétique, pharmaceutique ou dermatologique selon l'une quelconque des revendications 28 à 30, **caractérisée en ce qu'**elle est adaptée à une application par voie topique.

32. Composition cosmétique, pharmaceutique ou dermatologique selon l'une quelconque des revendications 28 à 31, **caractérisée en ce qu'**elle comprend également au moins un composé choisi parmi un agent émollient ou humectant, un agent gélifiant et/ou épaississant, un agent tensioactif, une huile, un agent actif, un colorant, un conservateur, un agent antioxydant, un agent chélatant, une poudre organique ou inorganique, un pigment, un agent autobronzant, un filtre solaire, une huile essentielle et un parfum.

33. Composition cosmétique, pharmaceutique ou dermatologique selon la revendication 32, **caractérisée en ce qu'**elle comprend un agent actif d'origine naturelle, biotechnologique ou synthétique ayant une activité biologique et ayant une efficacité sur la peau via des sites biologiques.

34. Composition cosmétique, pharmaceutique ou dermatologique selon l'une des revendications 32 ou 33, **caractérisée en ce que** ledit agent actif est choisi parmi les vitamines, les oligoéléments, l'allantoïne, les protéines végétales, les extraits végétaux et leurs mélanges.

35. Composition cosmétique, pharmaceutique ou dermatologique selon l'une des revendications 32 à 34, **caractérisée en ce que** ledit agent actif est un extrait d'algue.

36. Composition cosmétique, pharmaceutique ou dermatologique selon l'une quelconque des revendications 28 à 35, **caractérisée en ce qu'**elle se présente sous la forme d'une poudre, d'une émulsion, d'une microémulsion, d'une nanoémulsion, d'une suspension, d'une solution, d'une lotion, d'une crème, d'un gel aqueux ou hydroalcoolique, d'une mousse, d'un sérum, d'une solution ou d'une dispersion pour aérosol, ou d'une dispersion de vésicules lipidiques.
